# EUROPÄISCHE PATENTSCHRIFT

(11) **EP 3 354 213 B1**
(45) Veröffentlichungstag und Bekanntmachung des Hinweises auf die Patenterteilung: **05.05.2021**
(21) Anmeldenummer: 17000123.4
(22) Anmeldetag: 25.01.2017
(51) Int. Cl.: A61M 11/00, A61B 17/34, A61M 11/02, A61M 13/00

(54) **APPLIKATOR ZUM EINBRINGEN EINER SUBSTANZ IN EINE KÖRPERHÖHLE**
APPLICATOR FOR INTRODUCING A SUBSTANCE INTO A BODY CAVITY
APPLICATEUR DESTINÉ À INSÉRER UNE SUBSTANCE DANS UNE CAVITÉ CORPORELLE

(43) Veröffentlichungstag der Anmeldung: 01.08.2018
(73) Patentinhaber: König, Silke, 31789 Hameln (DE)
(72) Erfinder: König, Silke, 31789 Hameln (DE)
(74) Vertreter: Kayser, Christoph

(56) Entgegenhaltungen:
- WO-A1-2013/046710
- US-A1- 2010 168 779
- US-A1- 2010 198 014
- US-A1- 2012 209 166

## Beschreibung

Applikator mit einer Trokarhülse, einem in der Trokarhülse ausgebildeten Durchgangsloch und mit einer in Betriebsfunktion in dem Durchgangsloch der Trokarhülse geführten Nadel, an deren distalem Endbereich eine Nadeldüse ausgebildet ist.

Ein solcher Applikator ist zum Beispiel aus DE 10 2012 104 629 A1, aus US 2010/0331766 A1 und aus US 2010/168779 A1 (Basis für den Oberbegriff des Anspruchs 1) bekannt, der bei einer Laparoskopie zum Einsatz kommt. Mit einem solchen Applikator wird über eine Gaszufuhr ein Hohlraum im Körper des Patienten gebildet und über die Nadeldüse ein Medikament in den Hohlraum, in die Nähe des vorgesehenen Wirkungsortes eingebracht.

In den Druckschriften des Standes der Technik umfasst der Applikator neben der Trokarhülse und der Nadel eine Nadelhülse, die zwischen Nadel und Trokarhülse angeordnet ist und unterschiedliche Funktionen übernimmt, z.B. Bildung von Anschlüssen, Dichtung, etc.. Eine solche Nadelhülse ist ein technisches Bauteil, dessen Verwendung in einem hochsensiblen Einsatzbereich einer besonderen Sorgfalt bedarf. So muss jedes verwendete Bauteil strenge Kriterien erfüllen, um in der Medizintechnik eingesetzt werden zu dürfen. Jede mechanische Verbindung bedarf einer großen Präzision hinsichtlich einer Übertragung von Bewegungen, Kräften etc. Die Summe der verwendeten Bauteile erhöht die Anforderungen an jedes Bauteil, damit das Endergebnis der Gesamtheit aller Bauteile diese strengen Anforderungen erfüllt.

Es hat sich nun für den Fachmann überraschend gezeigt, dass ein Applikator der eingangs genannten Art in der Laparoskopie eingesetzt werden kann und die strengen Anforderungen erfüllt, wenn auf die Dreiteiligkeit des Standes der Technik verzichtet wird.

Die Aufgabe der vorliegenden Erfindung ist daher, einen Applikator des Standes der Technik derart weiterzubilden, dass dieser bei gleichbleibender oder besserer Präzision technisch vereinfacht ist und die Steuerung einer Substanzmenge erleichtert.

Die Aufgabe wird erfindungsgemäß dadurch gelöst, dass die Nadel mit ihrem Außenumfang an einem Innenumfang des Durchgangsloches dicht aber gleitfähig angeordnet ist und die Nadeldüse wenigstens eine Düsenöffnung aufweist.

Mit dem Gegenstand der vorliegenden Erfindung kann nunmehr auf die Nadelhülse ganz verzichtet werden. Die Nadel selbst gleitet dicht sind in der Trokarhülse. Zudem weist die Nadeldüse nicht nur eine Düsenöffnung auf, wie dies im Stand der Technik der Fall ist, sondern eine zur Dosierung einer in die Körperhöhle einzubringenden Substanz geeignete Düsenöffnung. Erstmals kann also nicht nur eine Substanz in die Körperhöhle in Wirknähe eingebracht sondern dort auch dosiert abgegeben werden.

Ein weiterer Vorteil der vorliegenden Erfindung ist, dass die Nadeldüse an dem distalen Endbereich der Nadel einen Düsenkopf bildet, der lösbar angebracht ist. Mit einem lösbaren Düsenkopf am distalen Endbereich der Nadel kann ein Austausch des Düsenkopfes in Abhängigkeit von der in die Körperhöhle einzubringenden Substanz bzw. deren Dosierung erfolgen. Es ist also nicht mehr notwendig, den ganzen Applikator auszutauschen. Vielmehr reicht es aus, nur den entsprechenden Düsenkopf am distalen Endbereich der Nadel anzubringen bzw. unter sterilen Verhältnissen auszutauschen.

Ein weiterer Vorteil der vorliegenden Erfindung ist, dass die Düsenöffnung eine Abmessung hat, die abhängig von einer in die Körperhöhle einzubringenden Dosis der Substanz ist. Die Steuerung der Abgabe der in den Körperhohlraum einzubringenden Substanz erfolgt nicht mehr nur über ein externes Steuergerät, sondern auch über die Größe der wenigstens einen

### Düsenöffnung.

Ein weiterer Vorteil der vorliegenden Erfindung ist, dass die wenigstens eine Düsenöffnung eine Kontur hat, deren Geometrie abhängig von der in die Körperhöhle einzubringenden Substanz ist. Anstelle der Abmessung oder auch zusätzlich zu der Abmessung kann die Steuerung der Abgabe der Substanz bzw. deren Dosierung auch über die Geometrie der Kontur der wenigstens einen Düsenöffnung erfolgen. So sind zum Beispiel Düsenöffnungen denkbar, die eine mehreckige, ovale und/oder runde Kontur haben.

Weitere Vorteile der vorliegenden Erfindung ergeben sich aus den weiteren Merkmalen der Unteransprüche.

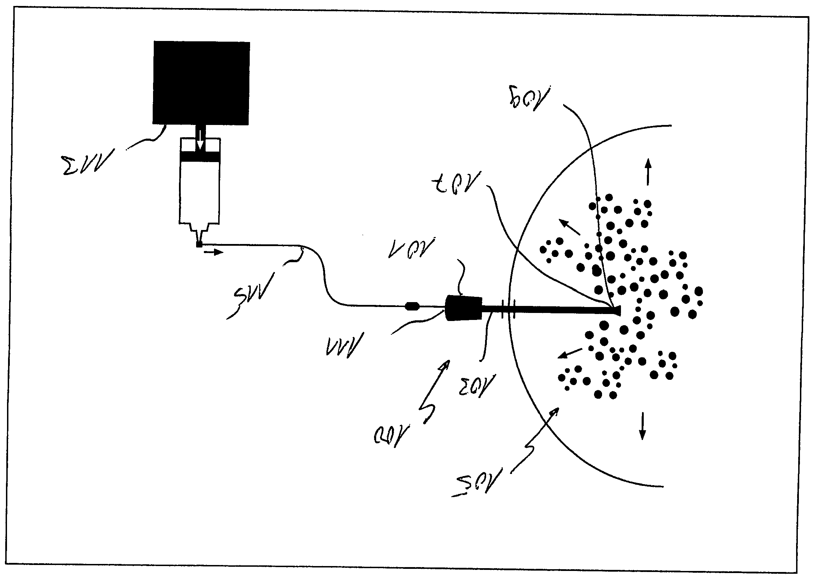

Eine Ausführungsform der Folge Erfindung wird im Folgenden anhand der einzigen Figur näher beschrieben.

Die Figur zeigt schematisch eine Anordnung 1 mit einem Applikator 100 gemäß der vorliegenden Erfindung.

Der Applikator 100 umfasst eine Trokarhülse 101, in der eine Nadel 103 geführt ist und in der Figur in einen schematisch dargestellten Körperhohlraum 105 eingedrungen ist. Ein distales Ende 107 liegt in dem Körperhohlraum 105 in der Nähe des gewünschten Wirkungsbereichs. In der Trokarhülse 101 ist eine Durchgangsöffnung (nicht dargestellt) ausgebildet, in der die Nadel 103 dicht und gleitfähig hin und her bewegt werden kann.

Das distale Ende 107 der Nadel 103 weist einen Düsenkopf 109 auf, der an dem distalen Ende 107 lösbar angebracht ist. Als lösbare Verbindung zwischen Düsenkopf 109 und distalem Ende 107 ist im Grunde jede beliebige mechanische Verbindung denkbar. Vorzugsweise ist der Düsenkopf 109 aber mithilfe einer Steckverbindung oder Schraubverbindung am distalen Ende 107 angebracht.

Denn Düsenkopf 109 weist wenigstens eine Düsenöffnung (nicht dargestellt) auf. Jede Düsenöffnung hat eine Abmessung und/oder Kontur, die eine Auswirkung auf eine Steuerung der Abgabe der in den Körperhohlraum einzubringenden Substanz hat. Die in den Körperhohlraum einzubringende Substanz soll an der Düsenöffnung mit einem Trägergas ein Aerosol bilden. Über die Größe und die Gestalt der wenigstens einen Düsenöffnung kann die Dosierung des Wirkstoffs in dem Aerosol gesteuert werden. An einem Düsenkopf 109 können demnach mehrere Düsenöffnungen mit unterschiedlichen Abmessungen und/oder Konturen vorgesehen sein. Die Wahl der Abmessung und der Kontur der wenigstens eine Düsenöffnung ist demnach abhängig von der in den Körperhohlraum einzubringenden Substanz.

Unterschiedliche Düsenköpfe 109 können sich in ihrer Anzahl von Düsenöffnungen und der Abmessung und/oder Kontur der wenigstens einen Düsenöffnung unterscheiden. Auf diese Weise kann der Operateur je nach Bedarf einen Düsenkopf 109 auswählen, der für den gerade vorliegenden Zweck geeignet ist.

Die Trokarhülse 101 weist einen Gasanschluss (nicht dargestellt) für eine Gasquelle auf, um den Körperhohlraum 105 mit einem Gas aufzublasen. Ein solches Gas ist vorzugsweise Kohlendioxid. Die Trokarhülse 101 weist auch einen Filteranschluss (nicht dargestellt) auf.

Die Trokarhülse 101 weist auch einen Anschluss 111 für eine mit einer Minipumpe 113 verbundenen Substanzquelle 114 auf. Die Substanzquelle 114 ist mit dem Anschluss 111 über einen Schlauch 115 verbunden. In den Schlauch 115 ist vorzugsweise in der Nähe des Anschlusses 111 ein Filterlement 116 lösbar oder nicht lösbar eingefügt. Ds filterlement 116 ist ein Feinfilter, um jederzeit die von der substanzquelle 114 für die jeweilige Substanz genannte Reinheit vor dem Eintritt in die Nadel 103 zu gewährleisten.

Über eine Steuerung der Minipumpe 113 lässt sich die in die Körperhöhle einzubringende Substanz über den Schlauch 115 in die Nadel 103 und die wenigstens eine Düsenöffnung in den Körperhohlraum abgeben. Neben der Steuerung der Minipumpe 113 bestimmt die wenigstens eine Düsenöffnung, bzw. bestimmen deren Abmessung und/oder Kontur die Dosierung der Substanz im Aerosol.

### Bezugszeichenliste

- 1: Anordnung
- 100: Applikator
- 101: Trokarhülse
- 103: Nadel
- 105: Körperhöhle
- 107: distales Ende
- 109: Düsenkopf
- 111: Anschluss
- 113: Minipumpe
- 114: Substanzquelle
- 115: Schlauch
- 116: Filterelement

## Patentansprüche

1. Applikator (100) zur Abgabe einer Substanz in eine Körperhöhle, mit einer Trokarhülse (101), einem in der Trokarhülse (101) ausgebildeten Durchgangsloch und mit einer in Betriebsfunktion in dem Durchgangsloch der Trokarhülse (101) geführten Nadel (103), an deren distalem Endbereich (107) eine Nadeldüse ausgebildet ist;
wobei die Nadeldüse wenigstens eine Düsenöffnung zur Abgabe einer in die Körperhöhle einzubringende Substanz aufweist; **dadurch gekennzeichnet,**
**dass** die Nadel (103) mit ihrem Außenumfang an einem Innenumfang des Durchgangsloches dicht aber gleitfähig angeordnet ist.

2. Applikator nach Anspruch 1,
**dadurch gekennzeichnet,**
**dass** die Nadeldüse an dem distalen Endbereich (107) der Nadel (103) einen Düsenkopf bildet, der lösbar angebracht ist.

3. Applikator nach Anspruch 2,
**dadurch gekennzeichnet,**
**dass** die wenigstens eine Düsenöffnung eine Abmessung hat, die abhängig von einer in die Körperhöhle einzubringenden Substanz ist.

4. Applikator nach einem der Ansprüche 1 bis 3,
**dadurch gekennzeichnet,**
**dass** die Trokarhülse (101) einen Gasanschluss aufweist.

5. Applikator nach einem der vorstehenden Ansprüche,
**dadurch gekennzeichnet,**
**dass** die Trokarhülse (101) einen Filteranschluss aufweist.

6. Applikator nach einem der vorstehenden Ansprüche,
**dadurch gekennzeichnet,**
**dass** der Applikator ein Pumpeinrichting umfasst, und
an einem proximalen Endbereich der Nadel (103) ein Anschluss (111) für die Pumpeinrichtung (113) ausgebildet ist, wobei die in die Körperhöhle (105) einzubringende Substanz von der Pumpeinrichtung (113) in die Nadel (103) und durch die Nadeldüse gepumpt werden kann.

7. Applikator nach Anspruch 6,
**dadurch gekennzeichnet,**
**dass** zwischen dem Anschluss (111) und der Pumpeinrichtung (113) ein Schlauch (115) angeordnet ist.

8. Applikator nach Anspruch 7,
**dadurch gekennzeichnet,**
**dass** in dem Schlauch (115) ein Filterelement (116) eingefügt ist.

9. Applikator nach einem der vorstehenden Ansprüche,
dass die in die Körperhöhle (105) einzubringende Substanz an der wenigstens einen Düsenöffnung in einem Aerosol enthalten ist.

10. Applikator nach einem der Ansprüche 2 bis 7,
**dadurch gekennzeichnet,**
**dass** der Düsenkopf mit dem distalen Endbereich (107) der Nadel (103) mechanisch verbunden ist.

11. Applikator nach Anspruch 8,
**dadurch gekennzeichnet,**
**dass** die Verbindung zwischen Düsenkopf und distalem Endbereich (107) der Nadel (103) eine Steckverbindung oder Schraubverbindung ist.

## Claims

1. An applicator (100) for delivery of a substance into a body cavity, with a trocar sleeve (101), a through-hole formed in the trocar sleeve (101), and with a needle (103), guided in operating function in the through-hole of the trocar sleeve (101), at the distal end region (107) of which needle a needle nozzle is formed;
wherein the needle nozzle has at least one nozzle opening for the delivery of a substance which is to be introduced into the body cavity;
**characterized in that**
the needle (103) is arranged with its outer circumference in a tight but slidable manner on an inner circumference of the through-hole.

2. The applicator according to Claim 1,
**characterized in that**
the needle nozzle forms at the distal end region (107) of the needle (103) a nozzle head, which is detachably mounted.

3. The applicator according to Claim 2,
**characterized in that**
the at least one nozzle opening has a dimension which is dependent on a substance which is to be introduced into the body cavity.

4. The applicator according to one of Claims 1 to 3,
**characterized in that**
the trocar sleeve (101) has a gas connection.

5. The applicator according to one of the preceding claims,
**characterized in that**
the trocar sleeve (101) has a filter connection.

6. The applicator according to one of the preceding claims,
**characterized in that**
the applicator comprises a pump arrangement, and
at a proximal end region of the needle (103) a connection (111) is formed for the pump arrangement (113), wherein the substance which is to be introduced into the body cavity (105) can be pumped by the pump arrangement (113) into the needle (103) and through the needle nozzle.

7. The applicator according to Claim 6,
**characterized in that**
between the connection (111) and the pump arrangement (113) a tube (115) is arranged.

8. The applicator according to Claim 7,
**characterized in that**
a filter element (116) is inserted in the tube (115).

9. The applicator according to one of the preceding claims,
**characterized in that**
the substance which is to be introduced into the body cavity (105) is contained at the at least one nozzle opening in an aerosol.

10. The applicator according to one of Claims 2 to 7,
**characterized in that**
the nozzle head is mechanically connected with the distal end region (107) of the needle (103).

11. The applicator according to Claim 8,
**characterized in that**
the connection between nozzle head and distal end region (107) of the needle (103) is a plug-in connection or screw connection.

## Revendications

1. Applicateur (100) pour libérer une substance dans une cavité corporelle, comprenant un manchon de trocart (101), un trou traversant formé dans le manchon de trocart (101) et comprenant une aiguille (103) dirigée en fonction d'utilisation dans le trou traversant du manchon de trocart (101) sur l'extrémité distale (107) de laquelle un injecteur d'aiguille est formé ;
dans lequel l'injecteur d'aiguille présente au moins une ouverture d'injecteur pour libérer une substance à introduire dans la cavité corporelle ;
**caractérisé en ce que**
l'aiguille (103) est disposée par son pourtour extérieur sur un pourtour intérieur du trou traversant, en étanchéité mais en pouvant glisser.

2. Applicateur selon la revendication 1,
**caractérisé en ce que**
l'injecteur d'aiguille forme une tête d'injecteur placée de manière séparable sur l'extrémité distale (107) de l'aiguille (103).

3. Applicateur selon la revendication 2,
**caractérisé en ce que**
l'au moins une ouverture d'injecteur a une dimension qui est fonction d'une substance à introduire dans la cavité corporelle.

4. Applicateur selon l'une des revendications 1 à 3,
**caractérisé en ce que**
le manchon de trocart (101) présente un raccordement pour du gaz.

5. Applicateur selon l'une des revendications précédentes,
**caractérisé en ce que**
le manchon de trocart (101) présente un raccordement pour un filtre.

6. Applicateur selon l'une des revendications précédentes,
**caractérisé en ce que**
l'applicateur comprend un dispositif de pompe, et
à une extrémité proximale de l'aiguille (103), un raccordement (111) pour le dispositif de pompe (113) est formé, dans lequel la substance à introduire dans la cavité corporelle (105) peut être pompée du dispositif de pompe (113) dans l'aiguille (103) et à travers l'injecteur d'aiguille.

7. Applicateur selon la revendication 6,
**caractérisé en ce qu'**
un tuyau (115) est disposé entre le raccordement (111) et le dispositif de pompe (113).

8. Applicateur selon la revendication 7,
**caractérisé en ce qu'**
un élément filtrant (116) est inséré dans le tuyau (115) .

9. Applicateur selon l'une des revendications précédentes,
**caractérisé en ce que**
la substance à introduire dans la cavité corporelle (105) est contenue dans un aérosol sur l'au moins une ouverture d'injecteur.

10. Applicateur selon l'une des revendications 2 à 7,
**caractérisé en ce que**
la tête d'injecteur est reliée mécaniquement à l'extrémité distale (107) de l'aiguille (103).

11. Applicateur selon la revendication 8,
**caractérisé en ce que**
la liaison entre la tête d'injecteur et l'extrémité distale (107) de l'aiguille (103) est un connecteur ou un raccord vissé.
